# EUROPEAN PATENT APPLICATION

(11) **EP 4 653 471 A1**
(43) Date of publication of application: **26.11.2025**
(21) Application number: 25178485.6
(22) Date of filing: 23.05.2025
(51) Int. Cl.: C08B 11/00, C08B 11/02

(54) **METHOD FOR PRODUCING LOW-SUBSTITUTED HYDROXYPROPYL CELLULOSE**

(30) Priority: 24.05.2024 JP 2024084436
(71) Applicant: SHIN-ETSU CHEMICAL CO., LTD.,, Chiyoda-ku, Tokyo 1000005 (JP)
(72) Inventor: HIRAMA, Yasuyuki, Niigata, 942-8601 (JP); INADA, Shinnosuke, Niigata, 942-8601 (JP)
(74) Representative: Ipsilon

(57) **Abstract**

Provided is a method for producing low-substituted hydroxypropyl cellulose having favorable disintegratability while maintaining sufficient compactibility. The method essentially includes the steps of: bringing an alkali metal hydroxide solution into contact with a pulp to prepare alkali cellulose; reacting the alkali cellulose with propylene oxide to obtain a reaction product; dispersing the reaction product into a solution containing at least an acid to partially solubilize the reaction product, and then neutralizing the reaction product with an additional amount of acid to precipitate crude low-substituted hydroxypropyl cellulose; using a screw press to remove liquid from the crude low-substituted hydroxypropyl cellulose to obtain purified low-substituted hydroxypropyl cellulose having a water content of 50 to 60% by mass; and drying and pulverizing the purified low-substituted hydroxypropyl cellulose, wherein the method produces low-substituted hydroxypropyl cellulose having a hydroxypropoxy group content of 5.0 to 16.0% by mass.

## Description

### BACKGROUND OF THE INVENTION

### Field of the invention

The invention relates to a method for producing low-substituted hydroxypropyl cellulose to be added as a disintegrant or a binder to a solid pharmaceutical preparation in the fields of pharmaceuticals or food, where the low-substituted hydroxypropyl cellulose exhibits good disintegratability.

### Background art

A solid pharmaceutical preparation as a pharmaceutical product, healthy food or the like is designed to disintegrate with the aid of a disintegrant contained therein that absorbs water and swells therein. Examples of the disintegrant include low-substituted hydroxypropyl cellulose, carboxymethyl cellulose and a calcium salt thereof, and starch and a derivative thereof.

Particularly in the field of pharmaceuticals, the number of newly developed drugs which lack stability is increasing and an available additive for use therein has also been limited from the viewpoints of their mutual action. Under these circumstances, the low-substituted hydroxypropyl cellulose (hereafter also referred to as "L-HPC") has been used widely as a nonionic disintegrant or a binder and can be regarded as a preferable additive.

A tablet, which is one of the dosage forms of the solid pharmaceutical preparation as a pharmaceutical product or food, is a solid pharmaceutical preparation formed by compressing powder into a predetermined shape. The tablet has advantages such as easy handleability. Particularly in the field of pharmaceuticals, the production amount of the tablet in monetary basis is about 50% of the total production amount of pharmaceuticals, thus making up the most commonly-used dosage form. The table can be produced by, for example, a dry direct tableting method, a dry granulation tableting method, an extrusion granulation tableting method, or a wet granulation tableting method.

As a method for producing L-HPC, there is known a method of controlling particles of fibrous form, the method including etherifying an alkali cellulose to complete etherification and then dispersing the reaction product of the etherification reaction in an aqueous solution containing an acid in such an amount as to be from 5 to 80% of the amount required for neutralizing the total amount of alkali to thereby dissolve a portion of L-HPC therein (See JP-A-S51-063927). The L-HPC produced by the above method is reported to have good compactibility and results in tablets having high degree of hardness when it is subjected to the dry direct tableting or wet granulation tableting method.

### SUMMARY OF THE INVENTION

However, when the L-HPC produced in accordance with a method described in JP-AS51-063927 is used for the wet granulation tableting method or the like, it has sufficient compactibility but may exhibit insufficient disintegratability. Thus, there is a demand for further improvement in disintegratability.

The invention has been made to overcome the drawback of the above-described prior art. An object of the present invention is to provide a method for producing L-HPC with good disintegratability while maintaining sufficient compactibility.

The inventors of the present invention diligently conducted a series of studies to address the aforementioned objectives and surprisingly found out that using a screw press in the production of L-HPC to remove liquid until the product reaches a water content of 50 to 60% by mass enhances swelling force and improves disintegratability without notably affecting its powder physical properties and compactibility, thus completing the invention.

The present invention provides a method for producing low-substituted hydroxypropyl cellulose that is as defined below:
<1> A method for producing low-substituted hydroxypropyl cellulose, comprising the steps of:
   bringing an alkali metal hydroxide solution into contact with a pulp to prepare alkali cellulose;
   reacting the alkali cellulose with propylene oxide to obtain a reaction product;
   dispersing the reaction product into a solution containing at least an acid to partially solubilize the reaction product, and then neutralizing the reaction product with an additional amount of the acid to precipitate crude low-substituted hydroxypropyl cellulose;
   using a screw press to remove liquid from the crude low-substituted hydroxypropyl cellulose to obtain purified low-substituted hydroxypropyl cellulose having a water content of 50 to 60% by mass; and
   drying and pulverizing the purified
   wherein the method produces low-substituted hydroxypropyl cellulose having a hydroxypropoxy group content of 5.0 to 16.0% by mass.
<2> The method according to <1>, wherein the screw press comprises a screw shaft having an inlet temperature of 100 to 160°C.

The L-HPC as obtained by the production method according to the present invention exhibits good disintegratability while maintaining sufficient compactibility, which allows a solid pharmaceutical preparation to have a shortened disintegration time and exhibit a rapid onset of action.

### BRIEF DESCRIPTION OF THE DRAWINGS

FIG. 1 is a schematic view illustrating an example of a screw press for use in a liquid removal step.
FIG. 2A is a front view of the schematic view illustrating an example of a tool for use in measuring swelling force.
FIG. 2B is a bottom view of the schematic view illustrating an example of a tool for use in measuring swelling force.
FIG. 3 is a schematic view illustrating a method for measuring swelling force using a tool as shown in FIGs. 2A and 2B.

### DETAILED DESCRIPTION OF THE INVENTION

The present invention will be explained in greater detail hereinbelow.

The method for producing L-HPC according to the present invention essentially includes the steps of: bringing an alkali metal hydroxide solution into contact with a pulp to prepare alkali cellulose; reacting the alkali cellulose with propylene oxide to obtain a reaction product; dispersing the reaction product into an aqueous solution containing at least an acid to partially solubilize the reaction product, and then neutralizing the reaction product with an additional amount of the acid to precipitate crude L-HPC; using a screw press to remove liquid from the crude L-HPC to obtain purified L-HPC having a water content of 50 to 60% by mass; and drying and pulverizing the purified L-HPC.

The term "screw press" as used herein refers to a machine for performing solid-liquid separation using a compressing force produced with the aid of variation in volume that varies from a supply region of the dewatering raw material to a discharge region of the dewatered product.

### [Step for preparing alkali cellulose]

First, in this step, a raw material pulp is brought into contact with an alkali metal hydroxide solution to prepare alkali cellulose. The alkali cellulose may be produced by immersing a sheet- or chip- shaped pulp in an alkali metal hydroxide solution and squeezing it off or by dropping or spraying an alkali metal hydroxide solution into powdery pulp for mixing the same in an internally stirring reactor.

Both the wood pulp and non-wood pulp such as linter pulp may be used but a wood-originating pulp is preferable from the viewpoint of using a GMO (Genetically Modified Organism)-free material. As the tree species of the wood, coniferous trees such as pine, spruce, and tsuga; and hardwood (broad-leaved) trees such as eucalyptus and maple may be used.

An alkali metal hydroxide solution of any type which is not particularly limited may be used so long as it enables pulp to turn into alkali cellulose but it is preferred from the viewpoint of economy that the solution be either of sodium hydroxide or potassium hydroxide. The concentration of the alkali metal hydroxide in the alkali metal hydroxide solution is preferably 20 to 60% by mass.

### [Step of reacting alkali cellulose with propylene oxide to obtain reaction product]

Next, in this step, the alkali cellulose prepared in the previous step is reacted with propylene oxide to obtain a reaction product. This step may be carried out by adding the alkali cellulose prepared in the previous step as well as propylene oxide into a reactor different from the above-mentioned rector to perform the reaction or by subsequently adding propylene oxide into the internally stirring reactor to perform the reaction if the same reactor has been used in the previous step. It is preferred that the amount of propylene oxide to be added thereinto be 0.05 to 0.5 parts by mass per 1 part by mass of anhydrous cellulose.

The propylene oxide may be added using any one of the methods of: adding a predetermined amount of propylene oxide all at once; adding a predetermined amount of propylene oxide in several batches; and adding a predetermined amount of propylene oxide continuously. The reaction temperature for this step is preferably 40 to 80°C. The reaction time of this step is preferably 1 to 5 hours. This step is preferably performed under a nitrogen atmosphere.

### [Precipitation step]

Next, in this step, the reaction product obtained in the previous step is first dispersed in an aqueous solution containing at least an acid and mixed therein to partially solubilize L-HPC. The amount of acid is preferably from 5 to 80%, more preferably from 10 to 60%, still more preferably from 10 to 40% of the amount (equivalent amount) required for neutralizing the alkali metal hydroxide present with the reaction product. Further, an additional amount of the acid is further added into the solution in which L-HPC is partially solubilized to completely neutralize the remaining alkali metal hydroxide, thereby precipitating crude L-HPC.

The equivalent amount required for neutralizing the alkali metal hydroxide present with the reaction product is an equivalent amount required for neutralizing the alkali metal hydroxide in the alkali metal hydroxide solution used for the contact with the pulp (This amount is hereafter also referred to simply as "neutralization equivalent"). Examples of acids for use therein include mineral acids, such as hydrochloric acid, sulfuric acid, and nitric acid, and organic acids such as formic acid and acetic acid, among which hydrochloric acid and acetic acid are preferred in terms of corrosiveness and toxicity.

### [Liquid removal step]

Next, in this step, a screw press is used to remove liquid from the crude L-HPC obtained in the previous step to obtain purified L-HPC.

It is preferred that the crude L-HPC obtained in the previous step be mixed with water before being subjected to this step to remove the byproducts produced up to and including the previous step. It is preferred in terms of extractability of byproducts such as salts and propylene glycol that the water to be mixed with the crude L-HPC have a temperature of 20 to 100°C, more preferably of 30 to 90°C. The mixture of water and the crude L-HPC may preferably be heated to 20 to 100°C, more preferably to 30 to 90°C after being mixed with each other.

Further, a washing step of washing the crude L-HPC obtained in the previous step to obtain washed L-HPC may be performed as necessary before being subjected to the liquid removal step. The washing step involves washing crude L-HPC by, for example, water using a continuous type horizontal vacuum filtration apparatus, a horizontal filter table filtration apparatus, or a horizontal belt filtration apparatus. The washed L-HPC may be mixed with water again to repeat the washing step multiple times. It is preferred for the purpose of the washing step that the water for use in the washing step have a temperature of 60 to 100°C.

It is preferred in terms of flowability that the mixture of water and the crude L-HPC before subjected to the liquid removal step have a water content of 80 to 98% by mass, more preferably 85 to 95% by mass.

The water contents of the crude L-HPC, washed L-HPC and purified L-HPC refer to values measured using a heat drying type moisture analyzer (MX-50 manufactured by A&D Company, Limited) under the conditions of a subject sample of 5 g, heating temperature at 105°C and heating time for 120 minutes. The water content as used herein is not a ratio of water to L-HPC but rather a content of water with respect to the total amount of L-HPC and water.

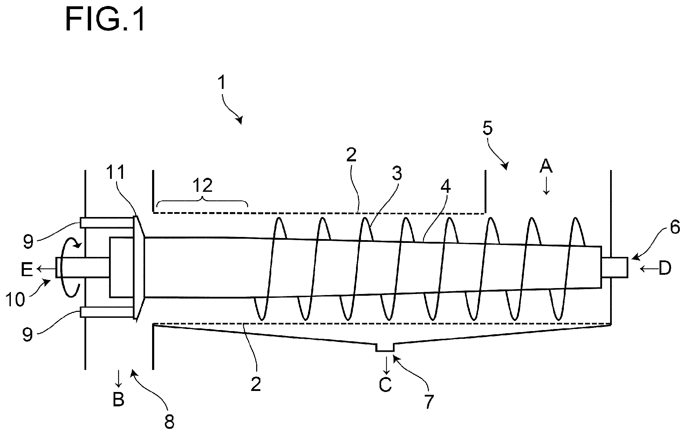

The screw press refers to a machine for performing solid-liquid separation using a compressing force produced with the aid of variation in volume that varies from a supply region of dewatering raw material toward a discharge region of dewatered product. FIG. 1 shows a schematic view illustrating an example of a screw press for use in the liquid removal step.

The screw press 1 is comprised of a substantially cylindrical filtration cylinder 2 having a plurality of pores formed by, for example, a punching process or slits for discharging a liquid component and a screw concentrically arranged inside the filtration cylinder 2, where the screw is comprised of a screw shaft 4 and a screw blade 3 welded thereon around the shaft 4. The screw shaft 4 has a volume that progressively expands from the supply region 5 of the dewatering raw material toward the discharge region 8 of the dewatered product. The screw 4 shaft allows a liquid or gas to run through from a temperature control liquid supplying port 6 to a temperature control liquid discharging port 10 to control the temperature. Further, the screw shaft 4 includes a straight region 12 where no screw blade 3 is provided at a section before the discharge region 8 of the liquid-removed product for the purpose of prolonging the residence time of the dewatering raw material A in the apparatus to enhance dewatering efficacy. That is, the dewatering raw material A, transported while being dewatered by volume variation from the supply region of the dewatering raw material toward the discharge region of the dewatered product, resides at the straight region 12 where no screw blade 3 is provided, which causes the dewatering raw material A, subsequently transported from the supply region 5 of the dewatering raw material, to be pressurized from the side of the discharge region 8 of the dewatered product, thus allowing a dewatered product B to further reduce its water content. The straight region 12 whose length is also referred to as the "straight length" or "plug length", can have its length adjusted by moving the screw that is parallelly movable along the main axis. The discharge region 8 of the dewatered product is provided with a back pressure plate 11 that has a shape of a circular truncated cone and is concentrically arranged around the screw shaft 4. The back pressure plate 11 allows for a controlled opening degree and can apply pressure (hereafter also referred to as "back pressure") using a pneumatic cylinder 9, from the discharge region 8 of the dewatered product toward the supply region 5 of the dewatering raw material. Pressate C is a liquid component squeezed off by the screw press 1 and passes through the pores in the filtration cylinder 2 and is discharged via a pressate discharging portion 7.

Examples of such screw press include an FKC screw press (manufactured by Fukoku Kogyo Co., Ltd.), an ISGK V-type hybrid pressure-fit screw press (manufactured by ISHIGAKI COMPANY, LTD), and a YSP type screw press dehydrator (manufactured by Yamato Sangyo Co., Ltd.).

A method for removing liquid from crude L-HPC or washed L-HPC using a screw press will be explained based on a screw press 1 as illustrated in FIG. 1.

First, a screw that intends to achieve a desired compression ratio is selected as appropriate. The term "compression ratio" as used with reference to a screw refers to the ratio of the cross-sectional area of the space between the inner wall of the filtration cylinder 2 and the screw shaft 4 at its smallest diameter to the cross-sectional area of the space between the inner wall of the filtration cylinder 2 and the screw shaft 4 at its largest diameter.

Next, the desired screw rotation speed, straight length, back pressure plate opening degree, back pressure, and screw shaft inlet temperature are set. After the setting is completed, the screw press 1 is put in operation to rotate the screw shaft 4. After the screw rotation speed reached a desired rotation speed, a mixture of water and the crude L-HPC or washed L-HPC is supplied thereinto as a dewatering raw material A from the supply region 5 of the dewatering raw material. The dewatering raw material A supplied from the supply region 5 of the dewatering raw material undergoes a gravity filtration through pores in the filtration cylinder 2 located immediately below the supply region 5 of the dewatering raw material A by which a liquid component is partially separated from solid. The dewatering raw material A from which a liquid component is partially separated is transferred by a rotating screw toward the discharge region 8 of the dewatered product. The liquid removal during this process is carried out by the variation in volume that varies from the supply region 5 of the dewatering raw material toward the discharge region 8 of the dewatered product. The dewatered product B is in the form of wet powder or wet aggregate which falls off from the discharge region 8 of the dewatered product by its own weight or collides with the back pressure plate 11 to be coarsely crushed and then falls off from the discharge region 8 of the dewatered product. The pressate C-a liquid component separated from solid-is discharged through pores in the filtration cylinder 2 and is collected from the pressate discharging portion 7.

It is preferred that the screw shaft have a screw shaft inlet temperature set at 100 to 160°C, more preferably at 110 to 150°C, and even more preferably at 115 to 145°C. A screw shaft inlet temperature below 100°C may fail to obtain purified L-HPC having a water content of 50 to 60% by mass, while a screw shaft inlet temperature above 160°C may cause the L-HPC to be dried on the screw shaft, adhered thereon, and solidified, and an over-dried product of adhering substance may be accidentally peeled off and mixed as an extraneous material into a manufactured product, thus risking a deterioration of the product.

It is preferred in terms of obtaining purified L-HPC having low water content that the compression ratio be 1.1 to 2.0, more preferably 1.2 to 1.8.

It is preferred in terms of obtaining purified L-HPC having low water content that the straight length be 100 to 150 mm, more preferably 120 to 150 mm.

It is preferred in terms of obtaining purified L-HPC having low water content that the back pressure be 0 to 0.5 MPa, more preferably 0.1 to 0.4 MPa.

The back pressure plate may be open to any extent which is not particularly limited but it is preferred in terms of dischargeability of the purified L-HPC that the back pressure plate be open to the extent of greater than 20 mm.

It is preferred in terms of obtaining purified L-HPC of low water content while maintaining a high level of production efficiency that the screw rotation speed be 0.5 to 6 rpm, more preferably 1 to 4 rpm.

The purified L-HPC obtained after the liquid removal step has a water content of 50 to 60% by mass, preferably of 50 to 57% by mass, and more preferably of 50 to 55% by mass. The purified L-HPC having a water content of less than 50% by mass results in a dewatered product which is excessively hard, and worsens its crushability, while the one having a water content above 60% by mass does not result in L-HPC with improved disintegratability.

### [Drying/Pulverizing step]

Finally, the purified L-HPC is dried and then pulverized to obtain L-HPC.

The drying may be performed using a dryer such as a shelf drier, a fluid-bed dryer, an agitated trough dryer, an agitated cylindrical dryer, or a steam tube rotary dryer.

The drying temperature is preferably 60 to 120°C, more preferably 80 to 100°C.

Pulverization may be carried out by using an impact-type pulverizer, such as a hammer mill, impact mill, or Victory mill, or by using a compaction pulverizer such as a roller mill or ball mill. Among them, an impact-type pulverizer is preferred in terms of energy efficiency. Moreover, it is preferred that the pulverized L-HPC be sieved using a known technique to remove insufficiently pulverized coarse powder. A sieve having an opening size of preferably 45 to 250 µm, more preferably 75 to 150 µm may be used.

### [Low-substituted hydroxypropyl cellulose (L-HPC)]

The degree of substitution of L-HPC obtained by the production method according to the present invention will be explained.

The hydroxypropoxy group content of L-HPC is 5.0 to 16.0% by mass, preferably 6.0 to 15.0% by mass, and more preferably 7.0 to 14.0% by mass. The L-HPC having a hydroxypropoxy group content of less than 5.0% by mass exhibits a low level of swelling property after water absorption, while the one having a hydroxypropoxy group content of higher than 16.0% by mass exhibits increased water solubility so that it leads to an insufficient level of disintegratability when used in a solid pharmaceutical preparation. The hydroxypropoxy group content of L-HPC may be measured by the assay described in "Low-Substituted Hydroxypropyl cellulose" of the Japanese Pharmacopoeia 18^{th} Edition.

It is preferred in terms of disintegratability and compactibility that the L-HPC have a volume-based median particle diameter, determined by dry laser diffraction method, of 10 to 100 µm, more preferably 30 to 80 µm, and even more preferably 40 to 70 µm. The term "median particle diameter" as used herein refers to a diameter corresponding to the 50% cumulative value on a volume-based cumulative distribution curve of the particles, which can be measured using, for example, a laser diffraction particle size analyzer of Mastersizer 3000 (manufacture by Malvern Panalytical Ltd.)

It is preferred in terms of flowability and compactibility that the L-HPC have a bulk density of 0.2 to 0.6 g/mL, more preferably 0.3 to 0.5 g/mL. The bulk density of L-HPC can be measured in accordance with the assay described in the third method (Measurement in a vessel) of "Measurement method of bulk density and tap density" 3.01 in General Test of the Japanese Pharmacopoeia 18th Edition. For example, a powder characteristics tester of POWDER TESTER PT-S type (manufactured by Hosokawa Micron Corporation) may be used for the measurement.

The L-HPC has a compactibility of preferably 100N or more, more preferably of 150N or more, from the viewpoint of tablet hardness or reduction of capping incidence.

The compactibility of the L-HPC refers to the hardness of a tablet formed by compressing 450 mg of the L-HPC whose humidity is controlled to have a loss on drying (i.e. water content) of 2.8 to 3.8% by mass, using a single-punch tableting machine with a round flat type punch having a diameter of 12 mm at a tableting pressure of 10.0 kN (about 88.5 MPa).

The loss on drying of L-HPC can be determined by the method described in "Loss on Drying test" in "General Tests" of the Japanese Pharmacopoeia 18th Edition.

The hardness of the tablet can be measured from the maximum strength at break at which the tablet is broken by a load applied at a predetermined rate in the diameter direction of the tablet. For example, it can be measured using a tablet hardness tester "TBH 125" (manufactured by ERWEKA GmbH).

It is preferred in terms of disintegratability that the L-HPC have a swelling force of 54N or greater, more preferably of 56N or greater. No particular limitation is therefore imposed on the upper limit, but it may be 100N. A tablet or granule with greater swelling force exhibits a shorter disintegration time and more rapid release of the drug, thus demonstrating better medical efficacy. The swelling force can be determined by using a texture analyzer of TA-XT plus (Stable Micro Systems, Ltd.). The details of the measurement will be explained in examples as described below.

Next, an example manner of producing tablets using L-HPC as a disintegrant by means of wet granulation tableting method will be explained. For example, L-HPC, an excipient such as lactose, a binder such as hydroxypropyl methylcellulose, and an active component such as a drug for use in pharmaceutical products are put into a wet agitation granulator to which water is then added for granulation, and then the mixture is dried using a fluid-bed dryer to produce a granulated product. Subsequently, the granulated product may be blended with a lubricant such as magnesium stearate, and then compressed at a predetermined pressure using a tablet machine, such as a rotary tablet machine, to produce tablets.

The median particle diameter of the granulated product varies depending on the application, but when used for tablets, it is preferably 60 to 300 µm, more preferably 70 to 200 µm, and even more preferably 80 to 150 µm, from the viewpoints of tablet compression and tablet mass variation. The median particle diameter of the granulated product may be determined by using a dry method based on the Fraunhofer diffraction theory with a laser diffraction particle size analyzer (Mastersizer 3000 produced by Malvern) under the conditions of dispersion pressure of 2 bar and scattering intensity of 2 to 10%, where a diameter corresponding to a 50% cumulative value on the volume-based cumulative particle diameter distribution curve may be used as the median particle diameter. It is preferred in terms of tablet compression and tablet mass variation that the granulated product have a bulk density of 0.2 to 0.7 g/mL, more preferably 0.3 to 0.6 g/mL. The bulk density of the granulated product can be measured in accordance with the assay described in the third method (Measurement in a vessel) of "Measurement method of bulk density and tap density" 3.01 in General Test of the Japanese Pharmacopoeia 18th Edition. For example, a powder characteristics tester of POWDER TESTER PT-S type (manufactured by Hosokawa Micron Corporation) may be used for the measurement.

It is preferred that the tablet have a hardness of 60 to 300N, more preferably of 80 to 200N, from the viewpoint of preventing, for example, cracking or chipping during packing, transporting, or removal of the tablet from a PTP sheet. The tablet hardness may be measured by using a tablet hardness meter (TBH 125 manufactured by ERWEKA GmbH) and be determined as the maximum breaking strength when a load is applied to the tablet in the diameter direction at a rate of 1 mm/sec until the tablet breaks.

The disintegration time of the tablet is preferably within 90 seconds, more preferably within 85 seconds, from the viewpoint of the onset of drug efficacy. The disintegration time of the tablet may be determined by using a disintegration tester (NT-400 produced by Toyama Sangyo Co., Ltd.) in accordance with the Disintegration Test (test solution: water, no supplementary plate) of the Japanese Pharmacopoeia 18th Edition.

### WORKING EXAMPLES

The present invention will be explained in detail with reference to working and comparative examples. It should not be construed that the invention is limited to or by the following examples.

### Working Example 1

Wood-originating sheet pulp was soaked in a sodium hydroxide aqueous solution having a concentration of 43% by mass at 35°C for 5 seconds, and then pressed to remove the excess aqueous sodium hydroxide solution to prepare alkali cellulose containing 22.0% by mass of sodium hydroxide (in which the mass ratio of sodium hydroxide relative to anhydrous cellulose in alkali cellulose was 0.479).

The resulting alkali cellulose was shredded by a slitter cutter. The shredded alkali cellulose, which was 100 parts by mass as anhydrous cellulose amount, was fed into an internally stirred pressurized reactor, and the nitrogen gas was thoroughly filled into the vessel of the reactor. After the nitrogen replacement, 27 parts by mass of propylene oxide was added to the reactor, and the contents of the reactor were reacted at 50°C for 3 hours to obtain a reaction product.

Then, 500 parts by mass of warm water at 45°C and 18.0 parts by mass of acetic acid (25% of the neutralization equivalent) were put into a kneader into which 100 parts by mass as anhydrous cellulose amount of the reaction product was dispersed and then mixed for 40 minutes (rotation speed: 43 rpm) at a jacket temperature of 45°C to dissolve a portion of the L-HPC. After that, 53.9 parts by mass of acetic acid (75% of the neutralization equivalent) was added thereinto to completely neutralize the solution to precipitate crude L-HPC.

Then, 1900 parts by mass of hot water at approximately 90°C was mixed with 100 parts by mass of the crude L-HPC, and the liquid was subsequently removed using a vacuum filtration apparatus. Subsequently, 1900 parts by mass of hot water at approximately 90°C was added thereinto and mixed again, and then the liquid was removed in a manner as explained above. This operation involving dispersion and liquid removal was repeated three times in total to wash the crude L-HPC and obtain washed L-HPC having a water content of 89.8% by mass.

Next, a screw press (SHX-200 manufactured by FKC Co., Ltd.) was used to remove liquid from the washed L-HPC under the conditions of: screw shaft inlet temperature of 120°C (with steam supplied at a vapor pressure of 0.1 MPa), compression ratio of 1.47, straight length of 130 mm, back pressure of 0.3 MPa, back pressure plate opening degree of 20 mm, and screw rotation speed of 2 rpm, thus resulting in purified L-HPC having a water content of 58.5% by mass.

After that, the purified L-HPC was dried at 80° C for 18 hours using a shelf-stage dryer, and the dried product was pulverized using an impact-type pulverizer (Victory Mill VP-1 manufactured by Hosokawa Micron) followed by being sieved through a sieve with an opening size of 90 µm to obtain L-HPC.

The resultant L-HPC underwent measurement of hydroxypropoxy group content, and the median particle diameter, bulk density, compactibility and swelling force thereof were measured in manners as described below. The results are as shown in Table 1.

### <Measurement of median particle diameter>

As for the median particle diameter, a diameter corresponding to the 50% cumulative value of the volume-based cumulative particle diameter distribution curve was measured under conditions of a dispersion pressure of 2 bar and a scattering intensity of 2 to 10% by the dry method according to the Fraunhofer diffraction theory using a laser diffraction particle size analyzer of Mastersizer 3000 (manufactured by Malvern Panalytical Ltd).

### <Bulk density measurement >

The bulk density of L-HPC was measured using a powder characteristics tester of POWDER TESTER PT-S type (manufactured by Hosokawa Micron Corporation) in accordance with the assay described in the third method (Measurement in a vessel) of "Measurement method of bulk density and tap density" 3.01 in General Test of the Japanese Pharmacopoeia 18th Edition.

### <Compactibility measurement>

The compactibility was determined by a process including the steps of: storing L-HPC in a desiccator (relative humidity: about 11%) of 25°C containing a saturated aqueous solution of lithium chloride for one week to control the water content to 2.8 to 3.8% by mass; compressing the stored L-HPC to form it into a 450 mg tablet at a tableting pressure of 10 kN (about 88.5 MPa) by using a table top type tablet forming press "HANDTAB-200" (manufactured by ICHIHASHI SEIKI KYOTO JAPAN) equipped with a round and flat punch having a diameter of 12 mm; and determining the hardness of the tablet as the maximum strength at break when a load was applied to the tablet at a rate of 1 mm/sec in the tablet diameter direction until the tablet was broken by using a tablet hardness tester "TBH 125" (manufactured by ERWEKA GmbH).

### <Swelling force measurement>

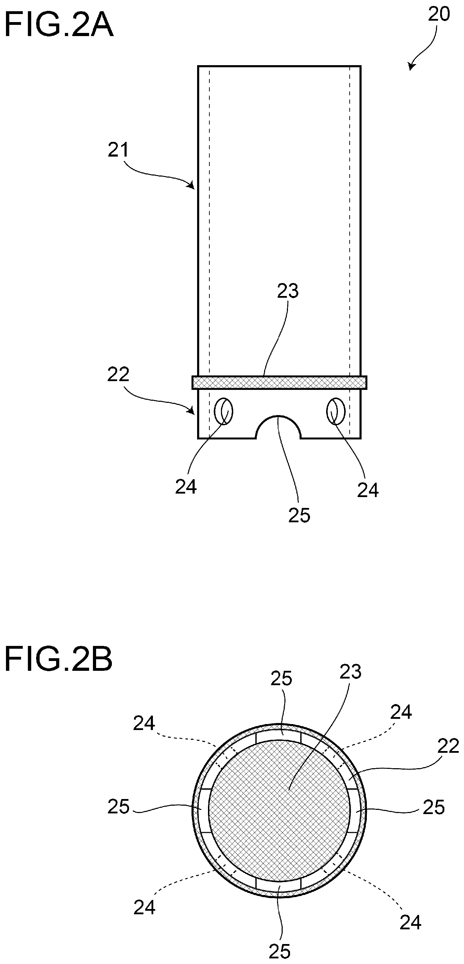

A swelling force measuring tool 20, as illustrated in FIGs. 2A and 2B and made by the applicant, was used for measuring the swelling force. The swelling force measuring tool was comprised of a first acrylic tube 21, a second acrylic tube 22, and a plain-woven wire mesh 23 of 18 mesh (opening size of 850 µm).

A first acrylic tube 21 having an inner diameter of 26 mm, an outer diameter of 30 mm, and a length of 60 mm was used. A second acrylic tube 22, having an inner diameter of 26 mm, an outer diameter of 30 mm, and a length of 10 mm, was used, where the tube was provided with four circular openings 24 each with a diameter of 4 mm on the lateral side, and four semicircular grooves 25, each with a diameter of 8 mm on the bottom surface, as shown in FIGs. 2A and 2B. A plain-woven wire mesh 23 of 18 mesh (with an opening size of 850 µm), cut into a circular shape with a diameter of 31 mm, was used.

The plain-woven wire mesh 23 of 18 mesh (with an opening size of 850 µm) was sandwiched by the first acrylic tube 21 and the second acrylic tube 22, which were joined by applying glue from outside to thereby make a swelling force measuring tool.

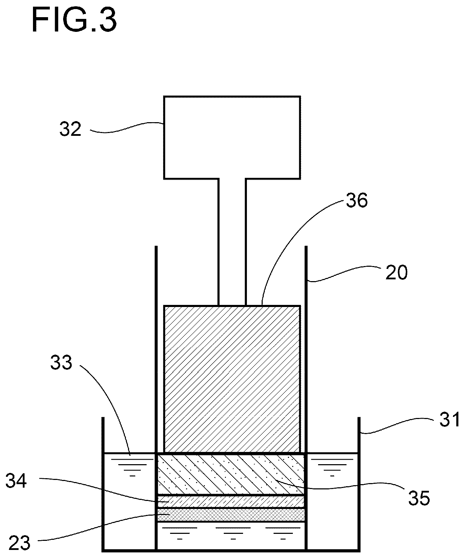

FIG. 3 illustrates a method for measuring swelling force using the swelling force measuring tool 20 shown in FIGs. 2A and 2B. A texture analyzer 32 of TA-XT plus (manufactured by Stable Micro Systems) was used for measuring the swelling force in which the swelling force measuring tool 20 was placed at the center of a stainless-steel petri dish 31 (with an outer diameter of 75 mm, an inner diameter of 74 mm, and a height of 20 mm). A filter paper 34 (with a diameter of 25.5 mm), a sample material 35 (1.00 g), and a measurement probe 36 (25 mm Cyl.Perspex) were set on the wire mesh 23 of the measuring tool 20. After applying a load of 1000 g three times to perform calibration, measurement was started using a measurement program of HLDD under the conditions of pretest speed of 2.0 mm/sec, test speed of 1.0 mm/sec, force of 20 g, and trigger force of 5 g. The sample material 35 and water 33 at 25°C were poured up to the point near the interface between the sample material 35 and the measurement probe 36. The time when the sample material began to absorb water was designated as 0 minutes, and the value after 10 minutes was determined as the swelling force.

The measurement was repeated three times, respectively, to calculate the average value and standard deviation.

The resultant L-HPC as obtained above was used to produce granulated products and tablets by wet stirring granulation tableting in a manner as explained below, and their physical properties were measured.

210 g (70 parts by mass) of acetaminophen (fine powder grade, manufactured by Yamamoto Kagaku Kogyo Co., Ltd.) as an active ingredient, 66 g (22 parts by mass) of lactose (Pharmatose 200M, manufactured by DFE Pharma) as an excipient, 15 g (5 parts by mass) of L-HPC as a disintegrant, and 9 g (3 parts by mass) of hydroxypropyl methylcellulose as a binder were put in a wet stirring granulator (VG-05, produced by Powrex Corporation), and preliminarily mixed for 1 minute at the main blade rotation speed of 450 rpm and the cross-screw rotation speed of 3000 rpm. Subsequently, 57 g (19 parts by mass) of water was added into the same granulator, which was kneaded for 5 minutes at a main blade rotation speed of 450 rpm and a cross screw rotation speed of 3000 rpm, and then sieved through a JIS test sieve with an opening size of 1 mm to obtain a wet granulated product. The wet granulated product was placed in a fluidized bed granulation dryer (Multiplex MP-01, produced by Powrex Corporation), dried under the conditions of an inlet air temperature of 80°C and an air flow of 0.6 to 0.8 m³/min until the outlet air temperature reached 45°C, and then sieved through a JIS test sieve with an opening size of 500 µm to obtain a granulated product. The resultant granulated product had a median particle diameter and a bulk density as shown in Table 1.

Next, 1.0 g (0.5 parts by mass) of magnesium stearate (vegetable grade, produced by Taihei Chemical Industry Co., Ltd.) as a lubricant product was further added and mixed into 200 g (100 parts by mass) of the resultant granulated product, which was tableted at a tableting pressure of 12.0 kN (about 239 MPa) and a tableting speed of 20 rpm using a rotary tablet press of VIRGO (manufactured by Kikusui Seisakusho & Co.) to produce tablets each having a diameter of 8 mm, a curvature radius of 12 mm and a tablet mass of 200 mg. Six tablets were taken from the resultant tablets and were each measured for tablet hardness and disintegration time, and their average values and standard deviations are as shown in Table 1.

### Working Example 2

L-HPC was produced in the same manner as in Working Example 1 except that the screw press operating conditions were changed to have a screw shaft inlet temperature of 133°C (with steam supplied at a vapor pressure of 0.2 MPa) to obtain purified L-HPC having a water content of 53.3% by mass. The obtained L-HPC was measured for hydroxypropoxy group content, median particle diameter, bulk density, compactibility, and swelling force in the same manner as in Working Example 1. The results are as shown in Table 1.

Then, the L-HPC obtained in the above manner was used to perform wet granulation in the same manner as in Working Example 1, and resulted in a granulated product. The resultant granulated product was measured for median particle diameter and bulk density in the same manner as in Working Example 1. The results are as shown in Table 1.

Subsequently, the granulated product obtained above was used to perform tableting in the same manner as in Working Example 1 to obtain tablets. The obtained tablets were measured for tablet hardness and disintegration time in the same manner as in Working Example 1. The results are as shown in Table 1.

### Working Example 3

L-HPC was produced in the same manner as in Working Example 2 except that the screw press operating conditions were changed to have a straight length of 150 mm to obtain purified L-HPC having a water content of 50.4% by mass. The obtained L-HPC was measured for hydroxypropoxy group content, median particle diameter, bulk density, compactibility, and swelling force in the same manner as in Working Example 1. The results are as shown in Table 1.

Then, the L-HPC obtained in the above manner was used to perform wet granulation in the same manner as in Working Example 1, and resulted in a granulated product. The resultant granulated product was measured for median particle diameter and bulk density in the same manner as in Working Example 1. The results are as shown in Table 1.

Subsequently, the granulated product obtained above was used to perform tableting in the same manner as in Working Example 1 to obtain tablets. The obtained tablets were measured for tablet hardness and disintegration time in the same manner as in Working Example 1. The results are as shown in Table 1.

### Working Example 4

100 parts by mass as anhydrous cellulose amount of wood-originating powder pulp was fed into an internally stirred pressurized reactor, and 75 parts by mass of sodium hydroxide aqueous solution having a concentration of 35% by mass was fed hereinto while mixing the mixture, and mixed for 30 minutes at a jacket temperature of 45°C to prepare alkali cellulose containing 15.0% by mass of sodium hydroxide (mass ratio of sodium hydroxide to anhydrous cellulose in the alkali cellulose was 0.281).

Next, the vessel was thoroughly filled with nitrogen gas, and then 19 parts by mass of propylene oxide was put therein to make it react for two hours while stirring the same at a jacket temperature of 60°C to obtain a reaction product.

Then, 350 parts by mass of warm water at 45°C and 12.6 parts by mass of acetic acid (30% of the neutralization equivalent) were put into a kneader into which 100 parts by mass as anhydrous cellulose amount of the reaction product was dispersed, and then mixed for 40 minutes at a jacket temperature of 45°C to dissolve a portion of the L-HPC. After that, 29.5 parts by mass of acetic acid (70% of the neutralization equivalent) was added thereinto to completely neutralize the solution to precipitate crude L-HPC.

Subsequently, the crude L-HPC was washed in the same manner as in Working Example 1 to obtain washed L-HPC having a water content of 89.0% by mass.

After that, the liquid was removed from the washed L-HPC in the same manner as in Working Example 2 to obtain purified L-HPC having a water content of 54.0% by mass, and then L-HPC was obtained in the same manner as in Working Example 1.

The resultant L-HPC underwent measurement of hydroxypropoxy group content, and measured for median particle diameter, bulk density, compactibility, and swelling force in manners as described below. The results are as shown in Table 1.

Then, the L-HPC obtained in the above manner was used to perform wet granulation in the same manner as in Working Example 1 except that 60 g of water (20 parts by mass) was added thereinto, and resulted in a granulated product. The resultant granulated product was measured for median particle diameter and bulk density in the same manner as in Working Example 1. The results are as shown in Table 1.

Subsequently, the granulated product obtained above was used to perform tableting in the same manner as in Working Example 1 to obtain tablets. The obtained tablets were measured for tablet hardness and disintegration time in the same manner as in Working Example 1. The results are as shown in Table 1.

### Comparative Example 1

L-HPC was produced in the same manner as in Working Example 1 except that a V-type disc press (Asahi Press C-25 manufactured by Asahi Koki Co., Ltd.) was used in place of a screw press to remove water from the washed L-HPC to obtain purified L-HPC having a water content of 77.9% by mass.

The obtained L-HPC was measured for hydroxypropoxy group content, median particle diameter, bulk density, compactibility, and swelling force in the same manner as in Working Example 1. The results are as shown in Table 1.

Then, the L-HPC obtained in the above manner was used to perform wet granulation in the same manner as in Working Example 4, and resulted in a granulated product. The resultant granulated product was measured for median particle diameter and bulk density in the same manner as in Working Example 1. The results are as shown in Table 1.

Subsequently, the granulated product obtained above was used to perform tableting in the same manner as in Working Example 1 to obtain tablets. The obtained tablets were measured for tablet hardness and disintegration time in the same manner as in Working Example 1. The results are as shown in Table 1.

### Comparative Example 2

L-HPC was produced in the same manner as in Working Example 1 except that the screw press operating conditions were changed to have a screw shaft inlet temperature of 90°C (hot water was supplied) and a screw rotation speed of 3 rpm to obtain purified L-HPC having a water content of 66.1% by mass. The obtained L-HPC was measured for hydroxypropoxy group content, median particle diameter, bulk density, compactibility, and swelling force in the same manner as in Working Example 1. The results are as shown in Table 1.

Then, the L-HPC obtained in the above manner was used to perform wet granulation in the same manner as in Working Example 4, and resulted in a granulated product. The resultant granulated product was measured for median particle diameter and bulk density in the same manner as in Working Example 1. The results are as shown in Table 1.

Subsequently, the granulated product obtained above was used to perform tableting in the same manner as in Working Example 1 to obtain tablets. The obtained tablets were measured for tablet hardness and disintegration time in the same manner as in Working Example 1. The results are as shown in Table 1.

| | Form of raw material pulp | Dehydrator | Water content of purified L-HPC | Physical properties of low-substituted hydroxypropyl cellulose | | | | | | Physical properties of granulated product | | Physical properties of tablets | | | |
|---|---|---|---|---|---|---|---|---|---|---|---|---|---|---|---|
| | | | | Hydroxypropoxy group content | Median particle diameter | Bulk density | Compactibility | Swelling force (average value) | Swelling force (standard deviation) | Median particle diameter | Bulk density | Tablet hardness (average value) | Tablet hardness (standard deviation) | Disintegration time (average value) | Disintegration time (standard deviation) |
| | - | - | %by mass | %by mass | µm | g/mL | N | N | N | µm | g/mL | N | N | sec. | sec. |
| Working example 1 | Sheet | Screw press | 58.5 | 11 | 62 | 0.33 | 172 | 55.2 | 0.8 | 111 | 0.45 | 90.4 | 3.3 | 88.2 | 1.6 |
| Working example 2 | Sheet | Screw press | 53.3 | 11 | 63 | 0.35 | 169 | 57.6 | 1.3 | 111 | 0.44 | 89.0 | 3.1 | 84.6 | 1.4 |
| Working example 3 | Sheet | Screw press | 50.4 | 11 | 64 | 0.35 | 170 | 58.5 | 0.6 | 113 | 0.44 | 89.7 | 2.8 | 82.3 | 1.5 |
| Working example 4 | Powder | Screw press | 54.0 | 11 | 63 | 0.36 | 167 | 57.1 | 2.1 | 110 | 0.44 | 88.6 | 3.0 | 84.9 | 1.8 |
| Comparative example 1 | Sheet | V-type disc press | 77.9 | 11 | 66 | 0.33 | 168 | 48.5 | 0.4 | 113 | 0.46 | 90.1 | 4.0 | 100.8 | 1.7 |
| Comparative example 2 | Sheet | Screw press | 66.1 | 11 | 65 | 0.34 | 170 | 52.7 | 1.2 | 115 | 0.46 | 91.6 | 2.2 | 93.2 | 2.1 |

The amounts of water added during wet stirring granulation were finely tuned for performing wet stirring granulation because it is preferred that the physical properties (median particle diameter and bulk density) of granulated products be uniform for accurately evaluating the performance of L-HPC as a disintegrant. Specifically, 19 parts by mass of water relative to 100 parts by mass of the prepared powder was added in each of Working Examples 1 to 3, while 20 parts by mass of water was added in each of Comparative Examples 1 and 2 and Working Example 4 to perform wet stirring granulation, thereby obtaining the respective granulated products with almost the same degrees of median particle diameter and bulk density.

The purified L-HPCs of Working examples 1 to 4 each having a water content of 50 to 60% by mass had the same degrees of hydroxypropoxy group content, median particle diameter, and bulk density with those of Comparative Examples 1 and 2 whose purified L-HPCs each having a water content exceeding 60% by mass. The purified L-HPCs exhibited favorable compactibility maintained at a value greater than 150N. Surprisingly, despite this, their swelling force increased to 54N or more. This increase in swelling force improved the disintegration time of tablets to 90 seconds or less even though they exhibited the same degrees of tablet hardness, thus demonstrating excellent disintegratability. No mechanism has yet been identified so far that clarifies the increase in swelling force.

In addition, Working Examples 1 to 3 demonstrate that a purified L-HPC with lower water content exhibits higher swelling force and results in shorter tablet disintegration time. Further, Working Example 4 that employed powder pulp as a raw ingredient demonstrates that a purified L-HPC having a water content of 50 to 60% by mass exhibits a swelling force of 54N or higher and a disintegration time within 90 seconds regardless of their form of raw material pulp.

## Claims

1. A method for producing low-substituted hydroxypropyl cellulose, comprising the steps of:
bringing an alkali metal hydroxide solution into contact with a pulp to prepare alkali cellulose;
reacting the alkali cellulose with propylene oxide to obtain a reaction product;
dispersing the reaction product into a solution containing at least an acid to partially solubilize the reaction product, and then neutralizing the reaction product with an additional amount of the acid to precipitate crude low-substituted hydroxypropyl cellulose;
using a screw press to remove liquid from the crude low-substituted hydroxypropyl cellulose to obtain purified low-substituted hydroxypropyl cellulose having a water content of 50 to 60% by mass; and
drying and pulverizing the purified low-substituted hydroxypropyl cellulose
wherein the method produces low-substituted hydroxypropyl cellulose having a hydroxypropoxy group content of 5.0 to 16.0% by mass.

2. The method according to claim 1, wherein the screw press comprises a screw shaft having an inlet temperature of 100 to 160°C.
